# EUROPEAN PATENT SPECIFICATION

(11) **EP 4 593 807 B1**
(45) Date of publication and mention of the grant of the patent: **04.11.2026**
(21) Application number: 23782493.3
(22) Date of filing: 27.09.2023
(51) Int. Cl.: A61K 9/26, A61K 31/495, A61P 25/24

(54) **PALATABLE ORODISPERSIBLE FORMULATION OF VORTIOXETINE**
WOHLSCHMECKENDE ORODISPERSIBLE FORMULIERUNG VON VORTIOXETIN
FORMULATION ORODISPERSIBLE DE VORTIOXÉTINE AU GOÛT AGRÉABLE

(30) Priority: 30.09.2022 GB 202214446
(43) Date of publication of application: 06.08.2025
(60) Divisional application: 26152293.2 / 4 755 376
(73) Proprietor: Alkaloid AD Skopje, 1000 Skopje (MK)
(72) Inventor: VANOVA NAKJINOVA, Nadica, 1000 Skopje (MK); JANEVSKA, Ana, 1000 Skopje (MK); PETRUSEVSKI, Vlado, 1000 Skopje (MK)
(74) Representative: Cleveland Scott York
(86) International application number: PCT/EP2023/076756
(87) International publication number: WO 2024/068758

(56) References cited:
- EP-A1- 4 026 539
- CN-A- 105 534 933
- CN-A- 109 589 314

## Description

### Summary

The present invention relates to a novel orodispersible formulation of vortioxetine, to methods of preparing such a formulation, and such a formulation for use in a method of treatment of a patient.

### Background of the Invention

Vortioxetine (also known as Futhiaxetine) is a multi-target antidepressant with both 5-HT3 receptor antagonism and 5-HT1A receptor agonism, and is used for the treatment of major depression. With its unique properties and strong affinity towards serotonin transporter, vortioxetine is a modulator and stimulator of serotonergic transmission. Vortioxetine is an antidepressant drug suitable for therapy in various types of depression: severe, anxiety-associated, and of elders. It acts equally as strongly as SNRIs or agomelatine and has favourable effects on cognitive functioning. At present, vortioxetine hydrobromide is used clinically as its active ingredient. The product name is Brintellix has four approved specifications, 5mg, 10mg, 15mg and 20mg, and the dosage form is film-coated tablets.

The chemical name of vortioxetine hydrobromide is: 1-[2-(2,4-dimethylphenylsulfanyl)phenyl]piperazine hydrobromide. The structure is shown below

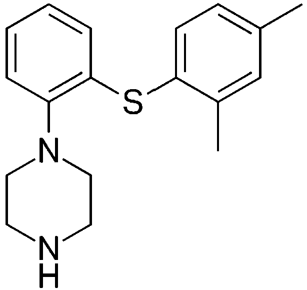

Vortioxetine is well-absorbed when taken orally and has an oral bioavailability of 75%. It is systemically detectable after a single oral dose by 0.781 hours. Peak levels of vortioxetine are reached within 7 to 11 hours post-dose with single or multiple doses. Steady-state levels of vortioxetine are generally reached within 2 weeks of administration, with 90% of individuals reaching 90% of steady state after 12 days of administration.

There is a clinical need for dosage forms to improve patients' medication compliance, particularly in the field of depression. Oral dispersible preparations are attractive, as they enable the drug to be easily dispersed and/or absorbed via the oral mucosa, and prevent issues such as patients hiding medications in the mouth, and subsequently disposing of them.

Due to the physical and chemical properties and clinical compliance of vortioxetine raw materials, few other dosage forms have been marketed so far.

Vortioxetine is very irritating to the oral mucosa, and caused discomfort to patients when administered directly to the mouth. A profound bitter, astringent, metallic taste can be appreciated even some days later. This affects compliance with the medication and limits the application of direct administration to the oral cavity. Therefore, it is necessary to prepare oral dispersible dosage forms other than film-coated tablets to overcome the problem of mucosal irritation.

Two main methods are used to overcome the unpleasant taste of drugs. The first method is to reduce the solubility of the drug and reduce the exposure of the drug to oral mucosa. Another method is to change the ability of the drug to interact with taste receptors, and affect the taste-related receptors, which will have potentially unknown side effects. Therefore, the first method is safer for patients. In order to reduce the irritation of the drug in the oral cavity, it is necessary to reduce the exposure of the drug in the oral cavity and reduce the dissolution of the drug; however, while reducing the dissolution of the drug, it will also slow down the absorption of the drug, thereby affecting the absorption and absorption of the immediate-release dosage form. The rate of metabolism affects the efficacy and safety of the drug. This technical contradiction is also the main technical obstacle for the product to market without oral taste-masking immediate-release dosage form. Especially when the patient's condition improves and is willing to cooperate with the medication to switch to the ordinary immediate-release coated tablets, the two dosage forms have different dissolution and absorption rates, which leads to potential physiological and pathological problems after the patient's dressing change.

The use of flavouring agents to adjust the taste is also a method to adjust the bitterness and astringency of the raw material, but the effect of the flavouring agent alone on the irritation improvement of vortioxetine is very poor.

Therefore, there is a need to develop new orodispersible formats of vortioxetine which address these and other problems.

### Prior Art

EP4026539A1 relates to an orally dispersible composition of vortioxetine hemihydrobromide, which comprises active ingredient and pharmaceutically acceptable excipients. The composition comprises solid dispersions of vortioxetine hemihydrobromide in taste masking excipients. The method of preparation involves powdering vortioxetine hemihydrobromide, coating (in a fluid bed) the vortioxetine hydrobromide powder with the taste masking excipients dispersed in a solvent, and removing the solvent to obtain the vortioxetine hemihydrobromide or vortioxetine hydrobromide coated with the taste masking excipient, and forming a suspension of vortioxetine hydrobromide.

US 2020/0188391A1 is aimed at solving the issue of poor solubility of vortioxetine hydrobromide and teaches that the particle size of vortioxetine hydrobromide as a critical measure for producing stable compositions of the active ingredient. It discloses pharmaceutical compositions containing vortioxetine hydrobromide having different particle size distributions.

### Summary of the Invention

In a first embodiment, the invention relates to an orodispersible formulation of vortioxetine comprising:
i. a granular component comprising vortioxetine hydrobromide together with calcium carbonate;
ii. a polymeric component selected from ethylcellulose, methylcellulose, a methacrylate based polymer, and combinations thereof;
iii. an extragranular matrix
wherein the polymeric component substantially encapsulates the granular component.

In a second embodiment, the invention relates to a method of preparing an orodispersible formulation according to the first embodiment, comprising steps of
i. granulating vortioxetine hydrobromide together with an inert carrier to form a granular component;
ii. coating the granular component of step i with a polymeric component;
iii. combining the coated granules of step ii with an extragranular matrix;
iv. forming the product of step iii into a solid oral dosage form.

In a third embodiment, the invention relates to an orodispersible formulation according to the invention for use in a method of treatment of a patient, wherein the method comprises direct administration of the orodispersible formulation to the oral mucosa, and allowing the formulation to dissolve.

### Brief Description of the Drawings

Figure 1 is a flow chart showing a process for the preparation of an orodispersible tablet according to an embodiment of the invention.
Figure 2 is a flow chart showing a process for the preparation of an orodispersible tablet according to an embodiment of the invention.

### Detailed description of the Invention

The problem of masking the unpleasant taste of vortioxetine has been addressed by the inventors by means of a novel formulation thereof, comprising granules of vortioxetine or a salt thereof together with an inert carrier (also referred to as the "granular component"), the granules being coated with a polymeric component, with the coated granules being embedded in a bulk extragranular matrix.

The present invention also provides a process for the preparation of the pharmaceutical composition, comprising a process step (i) forming vortioxetine and an inert carrier into granules; (ii) coating the granules with a polymer coating; (iii) mixing the coated granules with a bulk extragranular matrix; and (iv) forming the mixture into a solid dosage form.

The present invention also provides a pharmaceutical composition for use in the treatment of a disease selected from affective disorders, depression, major depressive disorder, postnatal depression, depression associated with bipolar disorder, Alzheimer's disease, psychosis, cancer, age or Parkinson's disease, anxiety, general anxiety disorder, social anxiety disorder, obsessive compulsive disorder, panic disorder, panic attacks, phobia, social phobia, agoraphobia, stress urinary incontinence, emesis, irritable bowel syndrome, eating disorders, chronic pain, partial responders, treatment resistant depression, Alzheimer's disease, cognitive impairment, attention deficit hyperactivity disorder, melancholia, posttraumatic stress disorder, hot flushes, sleep apnoea, alcohol, nicotine or carbohydrate craving, substance abuse and alcohol or drug abuse.

The compositions of the invention comprise vortioxetine hydrobromide salt.

Any form of vortioxetine hydrobromide can be used, for example crystalline vortioxetine hydrobromide, amorphous vortioxetine hydrobromide or mixtures thereof.

Suitable crystalline forms are e.g. the alpha, beta and gamma forms disclosed in WO2007/144005A1 or mixtures thereof. The free base of vortioxetine may be prepared as disclosed in WO2003/029232A1. Salts of vortioxetine may be prepared by dissolving the free base in an appropriate solvent, adding the relevant acid, followed by precipitation (the preparation of several solid forms of vortioxetine hydrobromide is disclosed e.g. in WO2007/144005A1).

Thus, the term granule, or granular component, as used herein, would include any suitable particulate or particle-like material. The granules may be of any appropriate shape, such as substantially spherical, prolate ellipsoid, sphero-cylinder, circular cylinder, tri-axial ellipsoid and hexagonal prism.

In accordance with an aspect of the granules have a desired, generally narrow, particle size distribution. The distribution range of the premix granules may be from at least 400, at least 600, or at least 630 microns to at most 2000, at most 1700, or at most 1530 microns. For example, the distribution range may be 400 to 2,000 microns, 600 to 1700 microns, or 630 to 1530 microns.

The desired sizes are obtained by screening, for example. The sizes correspond to granules that can or cannot pass through openings in industrial sieves or screens. Generally, the minimum particle size of the granules is large enough to eliminate the dust. The maximum particle size is generally selected to optimize appearance and maintain an acceptable rate of dissolution of the product.

The granules of the invention comprise vortioxetine in admixture with an inert carrier which comprises calcium carbonate. The inert carrier comprises one or more pharmaceutical excipients or adjuvants. Preferably, the vortioxetine salt is present in the granules in an amount of from 1 to 20 % w/w based on the weight of vortioxetine free base. More preferably, the vortioxetine salt is present in the granules in an amount of from 5 to 15 % w/w, such as about 10 % w/w.

The inert carrier may comprise one or more components selected from the group consisting of diluents, binders, bioadhesives, lubricants, disintegrants, preservatives, and flavouring agents.

Examples of pharmaceutically acceptable diluents include, but are not limited to, confectioner's sugar, compressible sugar, dextrates, dextrin, dextrose, lactose, mannitol, maize starch, microcrystalline cellulose, powdered cellulose, sorbitol, sucrose, calcium carbonate, and talc, and combinations thereof. The diluent includes at least one inorganic component which is calcium carbonate (CaCOs).

Preferably, the diluent is present as the major component (by weight) of the granules. For example, the diluent can be present in an amount of greater that 50 % w/w, such as greater than 60 % w/w, or greater than 70 % w/w.

Binders are optionally present in the granular component, and ensure that the granules can be formed with the required mechanical strength. Examples of binders include, but are not limited to, starches, celluloses and derivatives thereof, sucrose, dextrose, corn syrup, polysaccharides, and gelatin. Examples of celluloses and derivatives thereof include for example, microcrystalline cellulose. Particularly preferred is microcrystalline cellulose, e.g., Avicel PH 102 from Du Pont (Wilmington, Delaware, USA). Preferably, the binder is present in the granules in an amount of from 10 to 20 % w/w, such as about 15 % w/w.

The presence of a bioadhesive component can assist in maintaining contact of the active ingredient with the oral mucosae, improving thereby the therapeutic effectiveness and allowing a reduction of the amount of the active ingredients. Examples of bioadhesive polymers include polysaccharides from mammalian tissues (dermatan sulfate and chondroitin sulfate) as well as the polysaccharides of vegetable origin, mostly from algae, such as alginic acid, gellan and other related mucopolysaccharides, cellulose and derivatives thereof, chitosan and chitin, polyethylene glycols, polyvinylpyrrolidone, polyvinylalcohol (PVA) and carbomers. A preferred bioadhesive component is a carbomer (Poly(acrylic acid)), such as Carbopol 71G from Lubrizol Advanced Materials, Inc. Cleveland, Ohio, USA. Preferably, the bioadhesive is present in the granules in an amount of from 10 to 60 % w/w, such as 25 to 50 % w/w.

Disintegrants are added to oral solid dosage forms to aid in their deaggregation. Preferred disintegrants are sodium starch glycolate, silica, (such as hydrophilic fumed silica), croscarmellose sodium and crospovidone or mixtures thereof. Polyplasdone XL 10 (Ashland Global Specialty Chemicals Inc., Wilmington, Delaware, U.S.) is a suitable crospovidone. Aerosil 200 (Evonik Industries AG, Essen, Germany) is a suitable silica. Preferably, the disintegrants are present in the granules in an amount of from 1 to 10 % w/w, such as 2 to 5 % w/w.

Lubricants may also be incorporated in the granule composition to prevent adhesion and aid in production. Magnesium stearate is suitable in this context, and may present in the granules of from 0.5 to 2 % w/w.

The granules may also comprise an antimicrobial component (preservative). Suitable substances include benzalkonium chloride, benzethonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercury acetate, phenylmercuric nitrate, potassium benzoate, potassium sorbate , propylparaben, sodium propylparaben, sodium benzoate, sodium dehydroacetate, sodium propionate, sorbic acid, and thimerosal. Sodium acetate is preferred. Preferably, the antimicrobial component is present in the granules in an amount of from 1 to 5 % w/w.

Advantageously, the orodispersible formulation of the invention may comprise sodium acetate. Unexpectedly, it has been found that the inclusion of this material reduces the bitter taste of the active ingredient ("bitter blocking agent"), and improves tolerability and patient compliance. Sodium acetate may be present in the granular component, the extragranular component, or both. Sodium acetate may be present in an amount of 0.5 to 10 % w/w, preferably 1 to 5 % w/w.

The granules may be prepared by wet or dry granulation techniques. The granules may be sieved to obtain the desired particle size distribution characteristics. These are referred to hereinafter as "uncoated granules".

The polymer coating substantially encapsulates the granules. The polymer coating significantly reduces the perception of the bitter taste of vortioxetine.

The a polymeric component comprises one or more polymers, selected from ethylcellulose, methylcellulose, a methacrylate based polymer, and combinations thereof; preferably ethylcellulose, methylcellulose, ethyl acrylate/ methyl methacrylate copolymer, and dimethylaminoethyl metacrylate/butyl metacrylate/ methyl metacrylate copolymer. The release-controlling polymer may be hydroxypropyl methylcellulose. The amount of polymer may vary from 1 to 20 % w/w, based on the weight of the uncoated particles, preferably 2 to 15 % w/w, more preferably from 5 to 10 % w/w.

The polymeric component may be applied by a number of methods, where the granules are to be coated. The solvent-evaporation method is applicable, for example. Generally, the polymeric component is dissolved or dispersed in a suitable solvent. Therefore, in one embodiment the solvent-evaporation process comprises the steps of dissolving or dispersing polymer coating in a protic solvent, an aprotic solvent, or a mixture of a protic solvent and an aprotic solvent, adding the uncoated granules, mixing, e.g. in a fluidized bed, and removing the solvent.

In an alternative embodiment, fluid bed spray coating may be employed. A solution of the polymer coating is dissolved or dispersed in a suitable solvent, and applied as a spray to a fluidized bed of uncoated granules, operating in either, top spray, bottom spray, or tangential spray mode.

Removing the solvent can be performed in different ways. For example, the mixture can be stirred at 900 rpm for at least 1 hour to evaporate the solvent. Depending on the solvent used, this can be carried out at room or elevated temperatures. It is also possible to remove the solvent under reduced pressure. Other solvent removing techniques are well known to those skilled in the art. Optionally, the resultant coated granules are then stored, e.g. for at least 24 hours, in desiccators in order to remove remaining traces of solvent. In one embodiment, the fluidized coating enables efficacious evaporation of residual solvents, and no separate solvent removal step is required.

Examples of suitable solvents are water, alcohols, aliphatic hydrocarbons or esters. Particularly preferred is water, for environmental, flammability and safety reasons.

Spray drying is also a common manufacturing method for application of the polymer coating. Suitable spray-drying techniques are described, for example, by K. Masters in "The Spray Drying Handbook", John Wiley & Sons, New York, 1984 and Remington's Pharmaceutical Sciences, edition 20, edited by A. R. Gennaro, Mack Publishing Co., 2000.

Generally, the a polymeric component is dissolved or dispersed in a suitable solvent. The solution is applied to the granules as a spray. Then, heat from a hot gas such as heated air or nitrogen is used to evaporate the solvent from the coated granules formed. Other spray-drying techniques are well known to those skilled in the art.

Therefore, in one embodiment, the spray-drying process comprises the steps of dissolving or dispersing polymer coating in a protic solvent, an aprotic solvent, or a mixture of a protic solvent and an aprotic solvent, adding uncoated granules, mixing, and spray-drying the solution.

Typical solvents are for example water, alcohols, aliphatic hydrocarbons or esters. Particularly preferred are ethanol, isopropanol, methanol, acetone, 2-butanone, ethyl acetate, butyl acetate, tetrahydrofurane and methylene chloride.

The extragranular matrix contains the granular component embedded within it, and disintegrates or dissolves rapidly in the mouth without chewing or water. The extragranular matrix preferably comprises one or more components selected from the group consisting of diluents, binders, bioadhesives, lubricants, disintegrants, preservatives, flavouring agents and colouring agents.

Examples of extragranular diluents include, but are not limited to, confectioner's sugar, starch, compressible sugar, dextrates, dextrin, dextrose, lactose, mannitol, microcrystalline cellulose, powdered cellulose, sorbitol, sucrose, calcium carbonate, and talc, and combinations thereof. A combination of starch and mannitol is preferred. It is advantageous that the extragranular diluent is readily soluble, and hence mannitol is preferred. Preferably, the diluent is present in the extragranular component in an amount of from 20 to 99 % w/w, preferably from 30 to 90 % w/w, more preferably from 40 to 70 % w/w, such as about 50 % w/w or 70 % w/w.

Extragranular binders may be included to ensure that the finished dosage (e.g. tablet) can be formed with the required mechanical strength. Examples of extragranular binders include, but are not limited to, starches, celluloses and derivatives thereof, sucrose, dextrose, corn syrup, polysaccharides, and gelatin. Examples of celluloses and derivatives thereof include for example, microcrystalline cellulose and hydroxypropyl cellulose. Particularly preferred is microcrystalline cellulose, e.g., Avicel PH 102 from Du Pont (Wilmington, Delaware, USA), or hydroxypropyl cellulose, preferably a low-substituted hydroxypropyl cellulose (e.g. L-HPC NBD 022, Shin-Etsu Chemical Co., Ltd. Japan). Preferably, the binder is present in the extragranular component in an amount of from 10 to 20 % w/w, such as about 15 % w/w. In some embodiments, however, no extragranular binder is added, and only directly compressible mannitol is used. This ensures adequate mechanical strength of tablets after compression and acts as a diluent which improves mouthfeel.

Disintegrants may be included in the extragranular matrix forms to aid in their deaggregation. Preferred extragranular disintegrants are sodium starch glycolate, croscarmellose sodium and crospovidone or mixtures thereof. Polyplasdone XL 10 (Ashland Global Specialty Chemicals Inc., Wilmington, Delaware, U.S.) is a suitable crospovidone. A preferred extragranular disintegrant is sodium starch glycollate. Preferably, the disintegrants are present in the extragranular matrix in an amount of from 2 to 20 % w/w, such as 5 to 15 % w/w.

The extragranular matrix may also comprise an antimicrobial component (preservative). Suitable substances include benzalkonium chloride, benzethonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercury acetate, phenylmercuric nitrate, potassium benzoate, potassium sorbate , propylparaben, sodium propylparaben, sodium benzoate, sodium dehydroacetate, sodium acetate, sodium propionate, sorbic acid, and thimerosal. Sodium acetate is preferred, although the main function in the compositions of the invention is as a bitter blocking agent. Preferably, the antimicrobial component is present in the extragranular matrix in an amount of from 1 to 5 % w/w.

A sweetening agent may be included in the extragranular matrix to further improve palatability and mask the taste of the active ingredient. Preferred sweetening agents are selected from acesulfame potassium, alitame, aspartame, dextrose, erythritol, fructose, glucose, lactitol, maltitol, maltose, mannitol, neohesperidin dihydrochalcone, neotame, saccharin, sodium cyclamate, sorbitol, sucralose, sucrose, tagatose, thaumatin, and xylitol. Sucralose is preferred. Preferably, the sweetening agent is present in the extragranular matrix in an amount of from 1 to 5 % w/w.

One or more flavouring agents may be included in the extragranular matrix to further improve palatability and mask the taste of the active ingredient. Said flavouring agent is selected from the group consisting of: peppermint, apple, menthol, artificial vanilla, cinnamon, lemon, grapefruit, banana, and cherry, both individual and mixed, and the like are contemplated. Preferably, the one or more flavouring agents are present in the extragranular matrix in an amount of from 1 to 10 % w/w.

Lubricants may also be incorporated in the extragranular matrix composition to prevent adhesion and aid in production. Magnesium stearate is suitable in this context, and may present in the granules of from 0.5 to 5 % w/w.

The coated granules are preferably combined with the extragranular matrix composition and mixed using any suitable means. Careful mixing is desirable so as to maintain the integrity of the polymer coating on the granules. A drum hoop mixer is suitable.

The pharmaceutical compositions of the present invention are preferably in solid oral dosage form. Solid oral dosage forms include, but are not limited to tablets, hard or soft capsules, caplets, lozenges, pills, mini-tablets, pellets, beads, granules (e.g. packaged in sachets), or powders. Compositions intended for oral use are prepared according to any method known in the art for the manufacture of pharmaceutical compositions.

The solid oral dosage forms contain coated granules and extragranular matrix in a weight ratio of from 3:1 to 1:3, preferably from 2:1 to 1:2, such as about 1:1.

Tablets may be either film coated or enteric coated according to methods known in the art. Tablets can be optionally coated with a functional or non-functional coating as known in the art. Examples of coating techniques include, but are not limited to, sugar coating, film coating, microencapsulation and compression coating. Types of coatings include, but are not limited to, enteric coatings, sustained release coatings, controlled-release coatings.

Anhydrous pharmaceutical compositions and dosage forms can also be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (e. g., vials), blister packs, and strip packs.

The invention provides a method of treatment of a disease selected from affective disorders, depression, major depressive disorder, postnatal depression, depression associated with bipolar disorder, Alzheimer's disease, psychosis, cancer, age or Parkinson's disease, anxiety, general anxiety disorder, social anxiety disorder, obsessive compulsive disorder, panic disorder, panic attacks, phobia, social phobia, agoraphobia, stress urinary incontinence, emesis, irritable bowel syndrome, eating disorders, chronic pain, partial responders, treatment resistant depression, Alzheimer's disease, cognitive impairment, attention deficit hyperactivity disorder, melancholia, posttraumatic stress disorder, hot flushes, sleep apnoea, alcohol, nicotine or carbohydrate craving, substance abuse and alcohol or drug abuse, the method comprising administering an effective and generally non-toxic amount of vortioxetine to a subject in an orodispersible pharmaceutical dosage form, for example in a dosage form as described above.

Any other disease or condition treatable or preventable by vortioxetine may similarly be addressed by means of the invention. The invention therefore extends to the use of vortioxetine in the manufacture of an orodispersible pharmaceutical formulation. The invention also extends to a pack comprising an orodispersible pharmaceutical dosage form of vortioxetine together with instructions to place the dosage form in a patient's mouth. Encompassed within the invention is also a method for preparing a packaged dosage form of vortioxetine, the method comprising bringing into association an orodispersible pharmaceutical dosage form of vortioxetine and instructions to place the dosage form in a patient's mouth. The instructions may for example be printed on packaging encompassing the dosage form when sold or dispensed, or may be on a product information leaflet or insert within the packaging.

### Examples

### Example 1

| | Ingredients | mg per tablet |
|---|---|---|
| Granular Component | Vortioxetine HBr | 25,4 |
| | Calcium carbonate | 140,0 |
| | Microcrystalline Cellulose PH 102 | 30,0 |
| | Magnesium Stearate | 2,0 |
| **Taste masked granules** | | **197,4** |
| Coating component | Polymethacrylate-based coating system (Eudragit NM30 D) | 20,0 |
| **Weight of coated granules** | | **217,4** |
| Extragranular component | Mannitol (Pearlitol SD 200) | 150,6 |
| | Sodium starch glycollate | 20,0 |
| | Sucralose | 4,0 |
| | Flavour (Menthol + Lemon) | 4,0 |
| Mixing of final blend | Mg- stearate | 4,0 |
| **Extragranular weight** | | **182,6** |
| **FINAL Tablet weight** | | **400,0** |
| ** On dried polymer basis | | |

Orodispersible tablets were manufactured according to the following procedure.

The granular component ingredients (Vortioxetine HBR, calcium carbonate, microcrystalline cellulose and magnesium stearate) were dry granulated in a Fitzpatrick Chilsonator IR 220 roller compactor (The Fitzpatrick Company, 613 Colby Drive, Waterloo, ON, N2V 1A1, Canada). The coating component (Eudragit NM30 D) was applied using fluid bed spray granulation (top spray) in an Oystar Hüttlin Mycrolab fluid bed dryer (Hüttlin GmbH, Hohe-Flum-Straße 42, 79650 Schopfheim, Germany). The coated granules were sieved in a Quadro Comil conical screen mill (Quadro Engineering Corp, 613 Colby Drive, Waterloo, ON, N2V 1A1, Canada) to achieve an average particle size of below 1575 µm.

Coated granules were combined with the extragranular component (mannitol, sodium starch glycollate, sucralose and flavours) in a drum hoop mixer (ERWEKA GmbH, Pittlerstraße 45, 63225 Langen, Germany). Magnesium stearate was incorporated by the same means.

Tablets of 400 mg weight were formed on a Korsch XL 100 rotary tablet press (KORSCH AG, Breitenbachstrasse 1, D - 13509 Berlin, Germany).

### Example 2 - Reference

| | **Ingredients** | **mg per tablet** |
|---|---|---|
| Granular Component | Vortioxetine HBr | 25,4 |
| | Carbomer Homopolymer Type A (Carbopol 71G) | 25,4 |
| | Purified water | Q.S. |
| **Taste masked granules** | | **50,8** |
| Extragranular component | Microcrystalline Cellulose PH 102 | 60,0 |
| | Mannitol (Pearlitol SD 200) | 257,2 |
| | Sodium starch glycollate | 20,0 |
| | Sucralose | 4,0 |
| | Flavour (Menthol + Lemon) | 2,0 |
| Mixing of final blend | Magnesium stearate | 6,0 |
| **Extragranular weight** | | **349,2** |
| **FINAL Tablet weight** | | **400,0** |

Orodispersible tablets were manufactured according to the following procedure.

Vortioxetine HBr, carbomer homopolymer type A (Carbopol 71G) and purified water were wet granulated together in a high shear mixer (DIOSNA Dierks & Söhne GmbH, Am Tie 23, 49086 Osnabrück, Germany), and dried in an Oystar Hüttlin Mycrolab fluid bed dryer (Hüttlin GmbH, Hohe-Flum-Straße 42, 79650 Schopfheim, Germany). The granules were sieved in a Quadro Comil conical screen mill (Quadro Engineering Corp, 613 Colby Drive, Waterloo, ON, N2V 1A1, Canada) to achieve an average particle size of below 1575 µm. Coated granules were combined with the extragranular componentmicrocrystalline cellulose, mannitol, sodium starch glycollate, sucralose, andflavours) in a drum hoop mixer (ERWEKA GmbH, Pittlerstraße 45, 63225 Langen, Germany)Magnesium stearate was incorporated by the same means.

Tablets of 400 mg weight were formed on a Korsch XL 100 rotary tablet press (KORSCH AG, Breitenbachstrasse 1, D - 13509 Berlin, Germany).

### Example 3 - Reference

| | Ingredients | mg per tablet |
|---|---|---|
| Granular Component | Vortioxetine HBr | 25,4 |
| | Mannitol powder | 60,0 |
| | Carbomer Homopolymer Type A (Carbopol 71G) | 25,4 |
| | Purified water | Q.S. |
| **Taste masked granules** | | **110,8** |
| Extragranular component | Microcrystalline Cellulose PH 101 or 102 | 60,0 |
| | Mannitol (Pearlitol SD 200) | 197,2 |
| | Sodium starch glycollate | 20,0 |
| | Sucralose | 4,0 |
| | Flavour (Menthol + Lemon) | 2,0 |
| Mixing of final blend | Magnesium stearate | 6,0 |
| **Extra granular weight** | | **289,2** |
| **FINAL Tablet weight** | | **400,0** |

Tablets were prepared according to the method of Example 2.

### Example 4

| | Ingredients | 4(A) mg per tablet |
|---|---|---|
| Granular Component | Vortioxetine HBr | 25,4 |
| | Mannitol (Pearlitol SD 200) | 30, |
| | Calcium carbonate | 140,0 |
| | Magnesium stearate | 2,0 |
| **Compacted granules** | | **197,4** |
| Coating component | Ethylcellulose aqueous dispersion Surelease (Ethocel 25 % solids) | 25 mg solids (100 mg suspension) |
| Extragranular component | Microcrystalline Cellulose PH 102 | 20,0 |
| | Mannitol (Pearlitol SD 200) | 119,6 |
| | Sodium starch glycollate | 28,0 |
| | Sucralose | 4,0 |
| | Flavour (Menthol +Cherry) | 4,0 |
| Mixing of final blend | Magnesium stearate | 2,0 |
| **Extragranular weight** | | **177,6** |
| **FINAL Tablet weight** | | **400,00** |

Tablets of 400 mg weight were prepared according to the method of Example 1.

### Example 5

| | Ingredients | mg per tablet |
|---|---|---|
| Granular Component | Vortioxetine HBr | 25,4 |
| | Mannitol (Pearlitol SD 200) | 30,0 |
| | Calcium carbonate | 100,0 |
| | Sodium starch glycollate | 10,00 |
| | Sodium acetate | 4,0 |
| | Magnesium stearate | 2,0 |
| **Compacted granules** | | **171,4** |
| Coating component | Poly (meth)acrylate polymer Eudragit E 12,5 % solution (w.p dilution ~ 1:0,9) | 25,0 |
| Extragranular component | Microcrystalline Cellulose PH 102 | 20,0 |
| | Mannitol (Pearlitol SD 200) | 140,6 |
| | Low-substituted hydroxypropyl cellulose (L-HPC NBD 022) | 15,0 g |
| | Sodium acetate | 4,0 |
| | Sodium starch glycollate | 10,0 |
| | Sucralose | 4,0 |
| | Flavour (Menthol +Cherry) | 8,0 |
| Mixing of final blend | Magnesium stearate | 2,0 |
| **Extragranular weight** | | **203,6** |
| **FINAL Tablet weight** | | **400,0** |

Tablets of 400 mg weight were prepared according to the method of Example 1.

### Example 6

| | Ingredients | mg per tablet |
|---|---|---|
| Granular Component | Vortioxetine HBr | 25,4 |
| | Microcrystalline cellulose (Avicel PH 102) | 30,0 |
| | Calcium carbonate | 100,0 |
| | Sodium starch glycollate | 10,0 |
| | Sodium acetate | 4,0 |
| | Magnesium stearate | 2,0 |
| **Compacted granules** | | **171,4** |
| Coating component | Poly (meth)acrylate polymer Eudragit E 12,5 % solution (w.p dilution) | 25,0 |
| Extragranular component | Microcrystalline Cellulose PH 102 | 20,0 |
| | Mannitol (Pearlitol SD 200) | 140,6 |
| | Low-substituted hydroxypropyl cellulose (L-HPC NBD 022) | 15,0 g |
| | Sodium acetate | 4,0 |
| | Sodium starch glycollate | 10,0 |
| | Sucralose | 4,0 |
| | Flavour (Menthol +Cherry) | 8,0 |
| Mixing of final blend | Magnesium stearate | 2,0 |
| **Extragranular weight** | | **203,6** |
| **FINAL Tablet weight** | | **400,0** |

Tablets of 400 mg weight were prepared according to the method of Example 1.

### Example 7

| | Ingredients | mg per tablet |
|---|---|---|
| Granular Component | Vortioxetine HBr | 25,4 |
| | Mannitol (Pearlitol SD 200) | 70,0 |
| | Calcium carbonate | 100,0 |
| | Crospovidone (Polyplasdone XL 10) | 5,0 |
| | Sodium acetate | 2,0 |
| | Magnesium stearate | 2,0 |
| **Compacted granules** | | **204,4** |
| Coating component | Ethylcellulose aqueous dispersion Ethocel (Surelease) On dry weight basis | 20,0 |
| | Methylcellulose aqueous dispersion Methocel E5-LV | 4,0 |
| | Purified water (80 g per 4g methocel) | |
| **Coating Weight** | | 24,0 |
| Extragranular component | Sodium Chloride | 6,0 |
| | Mannitol (Pearlitol SD 200) | 192,6 |
| | Sodium acetate | 4,0 |
| | Crospovidone (Polyplasdone XL 10) | 15,0 |
| | Sodium starch glycollate | 20,0 |
| | Croscarmellose Sodium (Ac-Di-Sol) | 17,0 |
| | Hydrophilic fumed silica (Aerosil 200) | 2,0 |
| | Sucralose | 4,0 |
| | Flavour (grapefruit +banana) | 8,0 |
| Mixing of final blend | Magnesium stearate | 3,0 |
| **Extragranular weight** | | 271,6 |
| **FINAL Tablet weight** | | 500,0 |

Tablets of 500 mg weight were prepared according to the method of Example 1.

### Example 8

| | Ingredients | mg per tablet |
|---|---|---|
| Granular Component | Vortioxetine HBr | 25,4 |
| | Microcrystalline cellulose (Avicel PH 102) | 30,0 |
| | Sodium starch glycollate | 10,0 |
| | Calcium carbonate | 100,0 |
| | Sodium acetate | 4,0 |
| | Magnesium stearate | 2,0 |
| **Compacted granules** | | **171,4** |
| Coating component | Poly (meth)acrylate polymer Eudragit E 12,5 % solution (w.p dilution ~ 1:0,9) | 25,0 |
| **Coating Weight** | | 25,0 |
| Extragranular component | Microcrystalline cellulose (Avicel PH 102) | 20,0 |
| | Mannitol (Pearlitol SD 200) | 140,6 |
| | Low-substituted hydroxypropyl cellulose (L-HPC NBD 022) | 15,0 |
| | Sodium acetate | 4,0 |
| | Sodium starch glycollate | 10,0 |
| | Sucralose | 4,0 |
| | Flavour (menthol + cherry) | 8,0 |
| Mixing of final blend | Magnesium stearate | 3,0 |
| **Extragranular weight** | | 203,6 |
| **FINAL Tablet weight** | | 400,0 |

Tablets of 400 mg weight were prepared according to the method of Example 1.

### Example 9

| | Ingredients | mg per tablet |
|---|---|---|
| Granular Component | Vortioxetine HBr | 25,4 |
| | Mannitol (Pearlitol SD 200) | 70,0 |
| | Calcium carbonate | 100,0 |
| | Crospovidone (Polyplasdone XL 10) | 5,0 |
| | Sodium acetate | 2,0 |
| | Magnesium stearate | 2,0 |
| **Compacted granules** | | 204,4 |
| Coating component | Ethylcellulose aqueous dispersion Surelease (Ethocel 25 % solids) | 20,0 |
| | Methylcellulose aqueous dispersion Methocel E5-LV | 4,0 |
| | Purified water | 80mg per 4 mg Methocel |
| **Coating Weight** | | 24,4 |
| Extragranular component | Sodium Chloride | 6,0 |
| | Mannitol (Pearlitol SD 200) | 189,6 |
| | Sodium acetate | 4,0 |
| | Crospovidone (Polyplasdone XL 10) | 15,0 |
| | Sodium starch glycollate | 20,0 |
| | Croscarmellose Sodium (Ac-Di-Sol) | 17,0 |
| | Hydrophilic fumed silica (Aerosil 200) | 2,0 |
| | Sucralose | 7,0 |
| | Flavour (grapefruit + banana) | 8,0 |
| Mixing of final blend | Magnesium stearate | 3,0 |
| **Extragranular weight** | | 271,6 |
| **FINAL Tablet weight** | | 500,0 |

Tablets of 500 mg weight were prepared according to the method of Example 1.

### Example 10

| **Manufacturing phase** | **Ingredients** | **% in formulation** | **Function** |
|---|---|---|---|
| Dry granulation | Vortioxetine HBr | 5.09 | Active ingredient |
| | Mannitol (Pearlitol SD 200) | 14.0 | Filler |
| | Calcium carbonate | 20.0 | Filler |
| | Crospovidone (Polyplasdone XL 10) | 2.0 | Disintegrant |
| | Sodium acetate | 0.4 | Bitter blocking agent |
| | Magnesium stearate | 0.4 | Lubricant |
| **Compacted granules** | **/** | **41.89** | / |
| Coating of granules | Eudragit E 12.5 % | 6.0 | Polymer for release control and taste mask |
| | Purified water (1:6 to Eudragit) | / | Solvent |
| **Coated granules weight** | / | **47.89** | / |
| Blend | Mannitol + Maize starch (Pearlitol Flash) or Mannitol (Pearlitol SD 200) | 29.61 | Filler |
| | Sodium acetate | 0.8 | Bitter blocking agent |
| | Crospovidone (Polyplasdone XL 10) | 5.0 | Disintegrant |
| | Sodium starch glycollate | 4.0 | Disintegrant |
| | Croscarmellose Sodium (Ac-Di-Sol) | 3.0 | Disintegrant |
| | Colloidal silicon dioxide (Aerosil 200) | 0.4 | Adsorbent, disintegrant |
| | Sucralose | 1.4 | Sweetening agent |
| | Flavour (banana+ lemon or banana + apple) | 5.0 | Flavouring agents |
| | Taste mask | 1.4 | Taste masking agent |
| | Pigment iron oxide | 0.2 | Colouring agent |
| Final blend | Magnesium stearate | 1.3 | Lubricant |
| **Extragranular weight** | / | **52.11** | / |
| **Final tablet weight** | 500 mg | **100** | / |

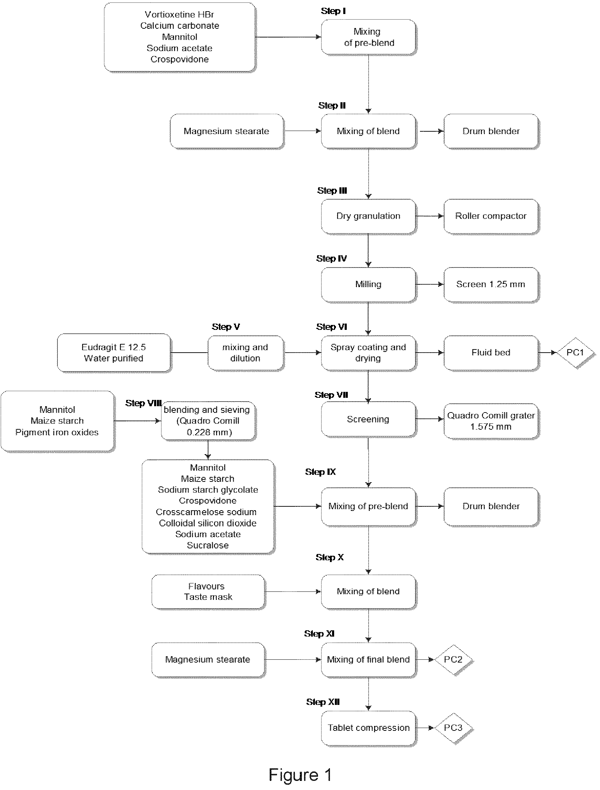

Tablets were prepared according to the process in Figure 1. "PC" in the diagram refers to process controls.

### Example 11

| **Manufacturing phase** | **Ingredients** | **% in formulation** | **Function** |
|---|---|---|---|
| Dry granulation | Vortioxetine HBr | 5.09 | Active ingredient |
| | Mannitol (Pearlitol SD 200) | 14.0 | Filler |
| | Calcium carbonate | 20.0 | Filler |
| | Crospovidone (Polyplasdone XL 10) | 2.0 | Disintegrant |
| | Sodium acetate | 0.4 | Bitter blocking agent |
| | Magnesium stearate | 0.4 | Lubricant |
| **Compacted granules** | **/** | **41.89** | |
| Coating of granules | Ethylcellulose aqueous dispersion Ethocel (Surelease) On dry weight basis | 5.0 | Polymer for release control and taste mask |
| | Methylcellulose aqueous dispersion Methocel E5-LV | 0.8 | Polymer for release control |
| | Purified water (1:20 to Methocel E5-LV) | / | Solvent |
| **coated granules weight** | **/** | **47.69** | / |
| Blend | Sodium Chloride | 1.3 | Bitter blocking agent and HPMC viscosity adjusting agent |
| | Mannitol + Maize starch (Pearlitol Flash) or Mannitol (Pearlitol SD 200) | 28.51 | Filler |
| | Sodium acetate | 0.8 | Bitter blocking agent |
| | Crospovidone (Polyplasdone XL 10) | 5.0 | Disintegrant |
| | Sodium starch glycollate | 4.0 | Disintegrant |
| | Croscarmellose Sodium (Ac-Di-Sol) | 3.0 | Disintegrant |
| | Colloidal silicon dioxide (Aerosil 200) | 0.4 | Adsorbent, disintegrant |
| | Sucralose | 1.4 | Sweetening agent |
| | Flavour (banana+ lemon or banana + apple) | 5.0 | Flavouring agents |
| | Taste mask | 1.4 | Taste masking agent |
| | Pigment iron oxide | 0.2 | Colouring agent |
| Final blend | Magnesium stearate | 1.3 | Lubricant |
| **Extragranular weight** | **/** | **52.31** | / |
| **Final tablet weight** | 500 mg | **100** | / |

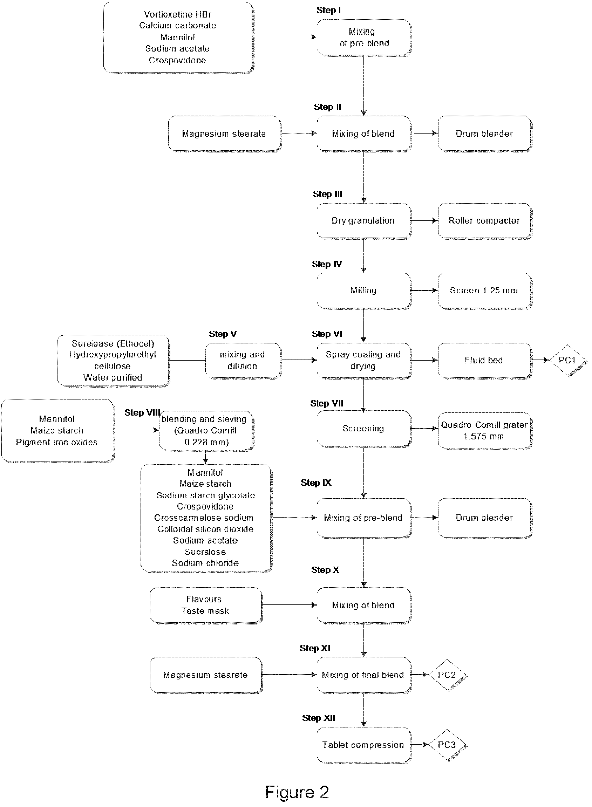

Tablets were prepared according to the process in Figure 2. "PC" in the diagram refers to process controls.

### Example 12

### Palatability Study

In order to compare the palatability of the compositions of the present invention with conventional compositions, a palatability study was conducted. A comparative composition (041221) was compared with two inventive compositions (260522 and 3849623).

Formulation 041221 (comparative) employed dry granulation with Eudragit EPO, Pearlitol SD 200 and Avicel PH 102 and direct compression of the active ingredient with external phase excipients including disintegrant + flavours + sweetener.

| **041221** | |
|---|---|
| **Ingredient** | **Qty in 400 mg tablets** |
| Vortioxetin HBr | 25.4 mg |
| Eudragit EPO | 20.0 mg |
| Magnesium stearate | 2.0 mg |
| MCC PH 102/Avicel | 60.0 mg |
| Pearlitol SD 200 | 262.5 mg |
| Sodium starch glycolate/Primojel | 20.0 mg |
| Sucralose | 4.0 mg |
| Flavour Menthol | 1.0 mg |
| Flavour Lemon | 1.0 mg |
| Magnesium stearate | 4.0 mg |

Formulation 260522 (according to the invention) employed dry granulation with calcium carbonate/Pearlitol SD 200/sodium acetate/Polyplasdone XL 10 and magnesium stearate and subsequent granule coating with Eudragit E 12.5 %, mixing with extragranular phase including disintegrants, flavours, and sweetener.

| **260522** | |
|---|---|
| **Ingredient** | **Qty in 500 mg tablets** |
| Vortioxetin HBr | 25.4 mg |
| Calcium carbonate | 100.0 mg |
| Pearlitol SD 200 | 70.0 mg |
| Sodium acetate | 2.0 mg |
| Polyplasdone XL 10 | 10.0 mg |
| Magnesium stearate | 2.0 mg |
| Eudragit E 12.5 % | 25 mg dry = 200 g solution |
| Pearlitol SD 200 | 179.6 mg |
| Sodium starch glycolate/Primojel | 20.0 mg |
| Polyplasdone XL 10 | 25.0 mg |
| Ac-Di-Sol | 15.0 mg |
| Aerosil 200 | 2.0 mg |
| Sucralose | 7.0 mg |
| Flavour grapefruit | 5.0 mg |
| Flavour banana | 5.0 mg |
| Bitter masking | 3.0 mg |
| Sodium acetate | 4.0 mg |

Formulation 3849623 (according to the invention) employed dry granulation with calcium carbonate/Pearlitol SD 200/Polyplasdone ultra and magnesium stearate and subsequent granule coating with Surelease -Ethylcellulose, and mixing with extragranular phase including disintegrants, flavours, and sweetener.

| **3849623** | |
|---|---|
| **Ingredient** | **Qty in 500 mg tablets** |
| Vortioxetin HBr | 25.4 mg |
| Calcium carbonate | 100.0 mg |
| Pearlitol SD 200 | 70.0 mg |
| Polyplasdone ultra | 10.0 mg |
| Magnesium stearate | 2.0 mg |
| Surelease (24.8 % solids) | 30 mg on dried = 121 g suspension |
| Methocel E5-LV in WP | 4.9 mg |
| Pearlitol SD 200 | 132.2 mg |
| Sodium starch glycolate/Primojel | 20.0 mg |
| Polyplasdone ultra | 7.5 mg |
| Ac-Di-Sol | 30.0 mg |
| Aerosil 200 | 1.5 mg |
| Avicel PH 101 | 25.0 mg |
| Sucralose | 6.0 mg |
| Flavor menthol | 20.0 mg |
| Sodium chloride | 6.0 mg |
| Sodium acetate | 3.0 mg |
| Sodium stearyl fumarate | 6.5 mg |

Volunteers were asked to read an information sheet regarding the properties of vortioxetine, and sampled formulations 041221, 260522 and 3849623 as described above. 20 mg tablets of formulations 041221, 260522 and 3849623 were assessed; half of one such tablet was used.

The following protocol was observed by the volunteers.

### Protocol for palatability assessment

1. A 20 mg round shaped biconvex tablet of the formulation under assessment was broken in half.
2. One half tablet was placed on the tongue and the mouth closed.
3. Once melted, the tablet was not swallowed; a sip of water was taken, and the mouth rinsed without swallowing, and the tablet spat out-;
4. 2 minutes were allowed to elapse after tasting and scoring, in order to be able to perceive the lingering palatability;

This procedure was repeated between each of the three different formulations tested.

Each formulation was ranked by participants in terms of overall palatability, mouth irritation and bitterness. Overall palatability was ranked using a modified Hedonic scale (Peryam 1957, Peryam 1952) from 1 (extremely unpleasant) to 9 (extremely pleasant). Mouth irritation was reported using a simple yes/no response. Bitterness was ranked from 1 (very bitter) to 3 (not bitter).

| **Formulation** | **Average taste result (modified Hedonic scale 1-extreme dislike-9 -like extremely)** | **Mouth irritation** | **Bitterness (1 -very bitter-3-not bitter)** |
|---|---|---|---|
| **041221** | 3.7 (moderate to slight dislike) | 83 % responded yes | 66.7 % responded very bitter |
| **260522** | 5.3 (neutral to like slightly) | 66.7 % responded no | 83.3 % responded somewhat bitter |
| **3849623** | 7.5 (moderate to liked very much) | 66.7 % responded no | 100 % responded not bitter |

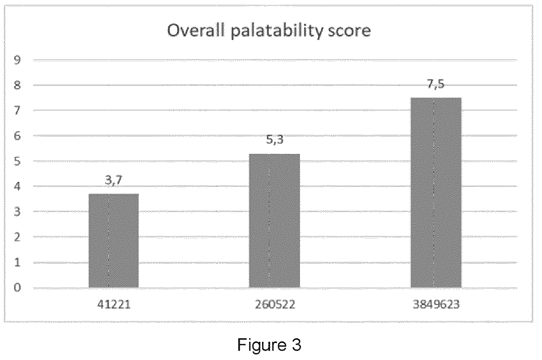

Overall palatability is shown in Figure 3.

Both of the inventive formulations showed clear superiority to comparative formulations in terms of overall taste, irritation and bitterness and ranked from neutral to likeable region of scoring compared to the formulation where the coating approach was not implemented.

When both the approaches are implemented (3849623), calcium carbonate granules containing API, polymer coating of the produced granules and flavouring/sweetener combination + bitter blocking by sodium acetate in the outer phase, the result was superior proving the efficacy of the combined taste masking approaches discussed in the current invention.

### Example 13

The inventors screened for the presence of N-nitroso vortioxetine impurity in the API. The latest EMA guidance stipulates acceptable intakes of selected nitrosamines as 400 ng/day.

Trials based on formulation 3849623 above comprising ascorbic acid (extra granularly), DL-α-tocopherol (added in the coating suspension) and BHA (extra granularly) in an amount of 0.05-3.5 wt %; propyl gallate may be optionally included. The levels of N-nitroso impurities are reduced.

## Claims

1. An orodispersible formulation of vortioxetine hydrobromide comprising:
i. a granular component comprising vortioxetine hydrobromide together with calcium carbonate;
ii. a polymeric component selected from ethylcellulose, methylcellulose, a methacrylate based polymer, and combinations thereof; and
iii. an extragranular matrix
wherein the polymeric component substantially encapsulates the granular component.

2. An orodispersible formulation according to claim 1 wherein the polymeric component comprises dimethylaminoethyl methacrylate/butyl methacrylate/ methyl methacrylate copolymer.

3. An orodispersible formulation according to claim 1 or 2 wherein the granules further comprise one or more of diluents, disintegrants, lubricants, preservatives, buffering agents, flavouring agents, bioadhesives, emulsifying agents, release-modifying agent, binders, viscosity-increasing agent and stabilizing agents.

4. An orodispersible formulation according to any preceding claim wherein the extragranular matrix comprises one or more of diluents, disintegrants, lubricants, preservatives, buffering agents, flavouring agents, bioadhesives, emulsifying agents, and binders.

5. An orodispersible formulation according to any preceding claim comprising an antioxidant preferably wherein the antioxidant is present in an amount of from 0.05 to 3.5 wt %.

6. An orodispersible formulation according to claim 5 wherein the antioxidant is present in the extragranular matrix, the polymeric composition, or both.

7. An orodispersible formulation according to any one of claims 5 to 6 wherein the antioxidant is selected from ascorbic acid, DL-α-tocopherol, butylated hydroxyanisole (BHA), propyl gallate and combinations thereof.

8. An orodispersible formulation according to any preceding claim in the form of a tablet.

9. An orodispersible formulation according to claim 8 wherein the vortioxetine is present in an amount of between 1 to 50 mg per tablet, preferably 5 to 25 mg, based on vortioxetine free base.

10. An orodispersible formulation according to claim 8 or 9 wherein the tablet has a weight of between 100 and 500 mg.

11. An orodispersible formulation according to any preceding claim for use in a method of treatment of a patient, wherein the method comprises direct administration of the orodispersible formulation to the oral mucosa, and allowing the formulation to dissolve.

12. An orodispersible formulation according to any preceding claim for use in a method of treatment of a disease selected from affective disorders, depression, major depressive disorder, postnatal depression, depression associated with bipolar disorder, Alzheimer's disease, psychosis, cancer, age or Parkinson's disease, anxiety, general anxiety disorder, social anxiety disorder, obsessive compulsive disorder, panic disorder, panic attacks, phobia, social phobia, agoraphobia, stress urinary incontinence, emesis, irritable bowel syndrome, eating disorders, chronic pain, partial responders, treatment resistant depression, cognitive impairment, attention deficit hyperactivity disorder, melancholia, posttraumatic stress disorder, hot flushes, sleep apnoea, alcohol, nicotine or carbohydrate craving, substance abuse and alcohol and drug abuse.

13. A method of preparing an orodispersible formulation as claimed in any one of claims 1 to 10 comprising steps of
i. granulating vortioxetine hydrobromide with an inert carrier to form a granular component;
ii. coating the granular component of step i with a polymeric component;
iii. combining the coated granules of step ii with an extragranular matrix;
iv. forming the product of step iii into a solid oral dosage form.

14. A method of claim 13 wherein the granulation step is a dry granulation.

15. A method of claim 13 or 14 comprising a further step of film coating the solid oral dosage form.

## Patentansprüche

1. Orodispersible Formulierung von Vortioxetin-Hydrobromid, die Folgendes umfasst.
i. eine granulare Komponente, die Vortioxetin-Hydrobromid zusammen mit Calciumcarbonat umfasst;
ii. eine polymere Komponente, die aus Ethylcellulose, Methylcellulose, einem Methacrylat-basierten Polymer und Kombinationen davon ausgewählt ist; und
iii. eine extragranulare Matrix,
wobei die polymere Komponente im Wesentlichen die granulare Komponente verkapselt.

2. Orodispersible Formulierung nach Anspruch 1, wobei die polymere Komponente Dimethylaminoethylmethacrylat/Butylmethacrylat/Methylmethacrylat-Copolymer umfasst.

3. Orodispersible Formulierung nach Anspruch 1 oder 2, wobei das Granulat weiter eines oder mehrere von Folgenden umfasst: Verdünnungsmitteln, Sprengmitteln, Schmiermitteln, Konservierungsmitteln, Puffern, Aromastoffen, Biohaftmitteln, Emulgatoren, Freisetzung modifizierendem Mittel, Bindemitteln, viskositätserhöhendem Mittel und Stabilisierungsmitteln.

4. Orodispersible Formulierung nach einem vorstehenden Anspruch, wobei die extragranulare Matrix eines oder mehrere von Folgenden umfasst: Verdünnungsmitteln, Sprengmitteln, Schmiermitteln, Konservierungsmitteln, Puffern, Aromastoffen, Biohaftmitteln, Emulgatoren und Bindemitteln.

5. Orodispersible Formulierung nach einem vorstehenden Anspruch, die ein Antioxidationsmittel umfasst, bevorzugt wobei das Antioxidationsmittel in einer Menge von 0,05 bis 3,5 Gew.-% vorliegt.

6. Orodispersible Formulierung nach Anspruch 5, wobei das Antioxidationsmittel in der extragranularen Matrix, der polymeren Zusammensetzung oder beidem vorliegt.

7. Orodispersible Formulierung nach einem der Ansprüche 5 bis 6, wobei das Antioxidationsmittel aus Ascorbinsäure, DL-α-Tocopherol, Butylhydroxyanisol (BHA), Propylgallat und Kombinationen davon ausgewählt ist.

8. Orodispersible Formulierung nach einem vorstehenden Anspruch in der Form einer Tablette.

9. Orodispersible Formulierung nach Anspruch 8, wobei das Vortioxetin in einer Menge von zwischen 1 bis 50 mg pro Tablette, bevorzugt 5 bis 25 mg, bezogen auf Vortioxetin als freie Base, vorliegt.

10. Orodispersible Formulierung nach Anspruch 8 oder 9, wobei die Tablette ein Gewicht von zwischen 100 und 500 mg aufweist.

11. Orodispersible Formulierung nach einem vorstehenden Anspruch für die Verwendung in einem Verfahren zur Behandlung eines Patienten, wobei das Verfahren Folgendes umfasst: direkte Verabreichung der orodispersiblen Formulierung an die Mundschleimhaut und Ermöglichen, dass sich die Formulierung auflöst.

12. Orodispersible Formulierung nach einem vorstehenden Anspruch für die Verwendung in einem Verfahren zur Behandlung einer Erkrankung, die aus Folgenden ausgewählt ist: affektiven Störungen, Depression, schwerer depressiver Störung, postnataler Depression, Depression im Zusammenhang mit bipolarer Störung, Alzheimer-Krankheit, Psychose, Krebs, Alter oder Parkinson-Krankheit, Angststörung, generalisierter Angststörung, sozialer Angststörung, Zwangsstörung, Panikstörung, Panikattacken, Phobie, sozialer Phobie, Agoraphobie, Belastungsinkontinenz, Erbrechen, Reizdarmsyndrom, Essstörungen, chronischen Schmerzen, partiellem Ansprechen, therapieresistenter Depression, kognitiver Beeinträchtigung, Aufmerksamkeitsdefizithyperaktivitätsstörung, Melancholie, posttraumatischer Belastungsstörung, Hitzewallungen, Schlafapnoe, Alkohol-, Nikotin- oder Kohlenhydratverlangen, Substanzmissbrauch und Alkohol- und Drogenmissbrauch.

13. Verfahren zur Herstellung einer orodispersiblen Formulierung nach einem der Ansprüche 1 bis 10, das die folgenden Schritte umfasst:
i. Granulieren von Vortioxetin-Hydrobromid mit einem inerten Träger, um eine granulare Komponente zu bilden;
ii. Beschichten der granularen Komponente von Schritt i mit einer polymeren Komponente;
iii. Vereinigen des beschichteten Granulats von Schritt ii mit einer extragranularen Matrix;
iv. Umformen des Produkts von Schritt iii in eine feste orale Dosierungsform.

14. Verfahren nach Anspruch 13, wobei der Granulierungsschritt eine Trockengranulierung ist.

15. Verfahren nach Anspruch 13 oder 14, das einen weiteren Schritt der Filmbeschichtung der festen oralen Dosierungsform umfasst.

## Revendications

1. Formulation orodispersible de bromhydrate de vortioxétine, comprenant :
i. un composant granulaire comprenant du bromhydrate de vortioxétine conjointement avec du carbonate de calcium ;
ii. un composant polymère choisi parmi l'éthylcellulose, la méthylcellulose, un polymère à base de méthacrylate, et des combinaisons de ceux-ci ; et
iii. une matrice extra-granulaire ;
dans laquelle le composant polymère encapsule sensiblement le composant granulaire.

2. Formulation orodispersible selon la revendication 1, dans laquelle le composant polymère comprend un copolymère de méthacrylate de diméthylaminoéthyle / méthacrylate de butyle / méthacrylate de méthyle.

3. Formulation orodispersible selon la revendication 1 ou 2, dans laquelle les granulés comprennent en outre un ou plusieurs parmi des diluants, des agents délitants, des lubrifiants, des conservateurs, des agents tampons, des agents aromatisants, des bioadhésifs, des agents émulsifiants, des agents modifiant la libération, des liants, des agents augmentant la viscosité et des agents stabilisants.

4. Formulation orodispersible selon l'une quelconque des revendications précédentes, dans laquelle la matrice extra-granulaire comprend un ou plusieurs parmi des diluants, des agents délitants, des lubrifiants, des conservateurs, des agents tampons, des agents aromatisants, des bioadhésifs, des agents émulsifiants et des liants.

5. Formulation orodispersible selon l'une quelconque des revendications précédentes, comprenant un antioxydant, préférablement où l'antioxydant est présent selon une quantité allant de 0,05 à 3,5% en poids.

6. Formulation orodispersible selon la revendication 5, dans laquelle l'antioxydant est présent dans la matrice extra-granulaire, la composition polymère, ou les deux.

7. Formulation orodispersible selon l'une quelconque des revendications 5 à 6, dans laquelle l'antioxydant est choisi parmi l'acide ascorbique, le DL-α-tocophérol, l'hydroxyanisole butylé (BHA), le gallate de propyle et des combinaisons de ceux-ci.

8. Formulation orodispersible selon l'une quelconque des revendications précédentes, sous la forme d'un comprimé.

9. Formulation orodispersible selon la revendication 8, dans laquelle la vortioxétine est présente selon une quantité allant d'entre 1 à 50 mg par comprimé, préférablement de 5 à 25 mg, sur la base de la vortioxétine sous forme de base libre.

10. Formulation orodispersible selon la revendication 8 ou 9, dans laquelle le comprimé présente un poids compris entre 100 et 500 mg.

11. Formulation orodispersible selon l'une quelconque des revendications précédentes, pour une utilisation dans une méthode de traitement d'un patient, la méthode comprenant l'administration directe de la formulation orodispersible sur la muqueuse buccale, puis laisser la formulation se dissoudre.

12. Formulation orodispersible selon l'une quelconque des revendications précédentes, pour une utilisation dans une méthode de traitement d'une maladie choisie parmi les troubles affectifs, la dépression, le trouble dépressif majeur, la dépression postnatale, la dépression associée au trouble bipolaire, la maladie d'Alzheimer, la psychose, le cancer, la vieillesse ou la maladie de Parkinson, l'anxiété, le trouble anxieux généralisé, le trouble d'anxiété sociale, le trouble obsessionnel-compulsif, le trouble panique, les crises de panique, la phobie, la phobie sociale, l'agoraphobie, l'incontinence urinaire d'effort, les vomissements, le syndrome du côlon irritable, les troubles alimentaires, la douleur chronique, les patients à réponse partielle, la dépression résistante au traitement, les troubles cognitifs, le trouble déficitaire de l'attention avec hyperactivité, la mélancolie, le syndrome de stress post-traumatique, les bouffées de chaleur, l'apnée du sommeil, l'envie d'alcool, de nicotine ou de glucides, l'abus de substances et l'abus d'alcool et de drogues.

13. Méthode de préparation d'une formulation orodispersible selon l'une quelconque des revendications 1 à 10, comprenant les étapes
i. de granulation de bromhydrate de vortioxétine avec un véhicule inerte pour former un composant granulaire ;
ii. d'enrobage du composant granulaire de l'étape i avec un composant polymère ;
iii. de combinaison des granulés enrobés de l'étape ii avec une matrice extra-granulaire ;
iv. de mise en forme du produit de l'étape iii en une forme posologique orale solide.

14. Méthode selon la revendication 13, dans laquelle l'étape de granulation est une granulation à sec.

15. Méthode selon la revendication 13 ou 14, comprenant une étape supplémentaire d'enrobage par film de la forme posologique orale solide.
